# DEMANDE DE BREVET EUROPEEN

(11) **EP 2 384 648 A1**
(43) Date de publication de la demande: **09.11.2011**
(21) Numéro de dépôt: 11164375.5
(22) Date de dépôt: 29.04.2011
(51) Int. Cl.: A23L 1/30, A61K 31/202, A61P 39/02, A61P 25/32, A23L 1/302, A23L 1/304

(54) **Nouvelle composition comprenant des oméga-3 et son utilisation dans la gueule de bois**

(30) Priorité: 07.05.2010 FR 1053598
(71) Demandeur: Deenox SAS, 75013 Paris (FR)
(72) Inventeur: Berthelemy, Olivier, 75013, PARIS (FR)
(74) Mandataire: Guérin, Jean-Philippe

(57) **Abrégé**

La présente invention concerne une nouvelle composition comprenant des oméga-3 et son utilisation dans la gueule de bois.

## Description

La présente invention concerne une nouvelle composition comprenant des oméga-3 et son utilisation dans la gueule de bois.

Les acides gras oméga-3, notés également ω₃ (ou encore n-3), sont des acides gras polyinsaturés que l'on trouve en grandes quantités dans certains poissons gras, le krill, le lin, la noix, la cameline et le colza.

Ce sont des acides gras essentiels qui sont utilisés principalement aujourd'hui pour la santé cardiovasculaire et la diminution du cholestérol.

La gueule de bois est une sensation inconfortable qui apparaît 6 à 8h après une consommation de boissons alcoolisée, lorsque l'alcoolémie diminue, et qui atteint un maximum lorsque l'alcaolémie redevient nulle (Prat G, Adan A, Sánchez-Turet M. Alcohol hangover: a critical review of explanatory factors. Hum Psychopharmacol 2009 Jun;24(4):259-67).

Selon une étude réalisée sur 1200 personnes (Slutske WS, Piasecki TM Hunt-Carter EE. Development and initial validation of the Hangover Symptoms Scale: prevalence and correlates of Hangover Symptoms in college students. Alcohol Clin Exp Res. 2003 Sep, 27(9):1442-50), les symptômes les plus fréquents sont la sensation de faiblesse (76%), la fatigue (73%), la difficulté de concentration (72%), les nausées (68%) et les maux de tête (68%).

La prévalence de la gueule de bois est grande :
- La consommation d'alcool est très répandue : moins de 15% des 15-64 ans sont abstinents en France (Com-Ruelle L, Dourgnon P. Jusot F, Latil E, Lengagne P. Identification et mesure des problèmes d'alcool en France : une comparaison de deux enquêtes en population générale. Questions d'économie de la santé ; 2005. 97:1-8).
- La gueule de bois peut apparaître dès la consommation d'un verre standard, et la consommation de 5 à 7 verres d'alcool est quasiment toujours suivie d'une gueule de bois (Wiese JG, Shlipak MG, Browner WS. The alcohol hangover. Ann Intern Med 2000 Jun 6;132(11):897-902).
- Il y a très peu de personnes « résistantes » à la gueule de bois. Plusieurs études ont permis de constater que seulement 20 à 30% des gens y sont insensibles (Howland J, Rohsenow DJ, Allensworth-Davies D, Greece J, Almeida A, Minsky SJ, Arnedt JT, Hermos J. The incidence and severity of hangover the morning after moderate alcohol intoxication. Addiction. 2008 May;103(5): 758-65).

Paradoxalement, la gueule de bois est plus fréquente chez les buveurs légers à modérés que chez les gros buveurs (Pristach CA, Smith CM, Whitney RB. Alcohol withdrawal syndromes - prediction from detailed medical and drinking histories. Drug Alcool Depend. 1983 Apr;11(2):177-99*;* Crofton J. Extent and costs of alcohol problems in employment: a review of British data. Alcohol Alcohol. 1987: 22(4):321-5), et les femmes y seraient plus sensibles (Smith CM, Barnes GM. Signs and symptoms of hangover: prevalence and relationship to alcohol use in a general adult population. Drug Alcohol Depend. 1983 Jun;11(3-4):249-69).

La gueule de bois a des conséquences économiques substantielles. Les études réalisées dans le monde mettent en évidence un coût important de la gueule de bois pour la société. Une grande perte économique a été identifiée au travail et en milieu universitaire à cause d'absentéisme, de baisse de productivité et de performance, d'accidents de travail et de conflits interpersonnels. L'absentéisme des jours suivant une consommation d'alcool est un des principaux facteurs du coût engendré.

L'origine de la gueule de bois n'a pas été encore complètement élucidée (Prat G, Adan A, sánchez-Turet M. Alcohol hangover: a critical review of explanatory factors. Hum Psychopharmacol. 2009 Jun;24(4):259-67).

Il apparaît cependant que la gueule de bois est un trouble complexe, pluri-factoriel et causé par 3 substances :
- l'éthanol, ou alcool pur, à l'origine de l'état d'ébriété
- l'acétaldéhyde, issu du métabolisme de l'éthanol
- les congénères, substances présentes dans les boissons alcoolisées, issues de la fermentation alcoolique ou ajoutées pendant le processus de production pour améliorer les propriétés organoleptiques - amines, amides, histamine, méthanol,...).

Jusqu'à présent, seuls quelques produits pour lutter contre la gueule de bois ont été commercialisés et de plus, peu d'entre eux ont montré une grande efficacité.

Ainsi, une étude sur un extrait d'artichaut (Pittler MH, White AR, Stevinson C, Ernst E. Effectiveness of artichoke extract in preventing alcohol-induced hangovers: a randomized controlled trial. CMAJ. 2003 Dec 9;169(12):1269-73) a mis en évidence l'absence d'efficacité dudit extrait sur la gueule de bois.

De même, l'huile de bourrache (Moesgaard et Hansen, 1992, PharmNord*, non publié*) n'a montré qu'une efficacité limitée sur quelques symptômes tels que les maux de tête, la paresse, la fatigue.

La figue de barbarie (Wiese J, McPherson S, Odden MC, Shlipak MG. Effect of Opuntia ficus indica on symptoms of the alcohol hangover. Arch Intern Med. 2004 Jun 28;164(12):1334-40) n'a démontré une efficacité significative que sur les nausées, l'anorexie et la bouche sèche mais le score total sur la gueule de bois n'a pas présenté de différence significative par rapport au placebo.

Un complément alimentaire Morning Fit^{®} (Laas I. A double-blind placebo-controlled study on the effects of Morning Fit on hangover symptoms after a high level of alcohol consumption in healthy volunteers. Journal of Drug Assessment 199;2:1-96) contenant de la levure (non identifiée) et des vitamines B a montré une amélioration dans les sensations subjectives telles que la sensation de se sentir mal, l'impatience et la fatigue.

L'acide tolfenamique (Kaivola S, Parantainen J, Osterman T, Timonen H. Hangover headache and prostaglandins: prophylactic treatment with tolfenamic acid. Cephalalgia. 1983 Mar;3(1):31-6), inhibiteur de l'action et de la synthèse des prostaglandines, a présenté une efficacité significative sur les maux de tête et le score total des symptômes.

Liv.52 (Chauhan BL, Kulkarni RD. Alcohol hangover and Liv. 52. Eur J Clin Pharmacol. 1991;40(2):187-8) est une préparation ayurvédique à base de plantes qui provoque une baisse significative des concentrations d'éthanol et d'acétaldéhyde dans le sang et les urines après 12h.

Le pyritinol (Encephabol®) (Khan MA, Jensen K, Krogh HJ. Alcohol-induced hangover. A double-blind comparison of pyritinol and placebo in preventing hangover symptoms. Q J Stud Alcohol. 1973 Dec;34(4):1195-201) dont la structure est apparentée à celle de la vitamine B6 sans en présenter les effets (notamment le métabolisme des lipides et des acides aminés), est un médicament neurotropique qui a également montré des effets significatifs sur la gueule de bois.

Enfin le magnésium *(*Altura BM, Altura BT. Association of alcohol in brain injury, headaches, and stroke with brain-tissue and serum levels of ionized magnesium: a review of recent findings and mechanisms of action. Alcohol. 1999 Oct;19(2):119-30) sous forme de MgSO₄, par voie intraveineuse, a montré un effet sur la gueule de bois sur un petit groupe de patients. Mais cette voie d'administration est difficilement envisageable par un particulier à domicile atteint de la gueule de bois.

Cependant, un article récent (Pittler MH, Verster JC, Ernst E. Interventions for preventing or treating alcohol hangover: systematic review of randomised controlled trials. BMJ. 2005 Dec 24; 331(7531):1515-8*.*) a conclu qu'il n'y avait pas de preuves irréfutable concernant les compositions ci-dessus citées, pour dire qu'elles puissent être efficaces dans le traitement ou la prévention de la gueule de bois.

Le but de l'invention est donc de fournir une composition alimentaire permettant de lutter efficacement contre la gueule de bois.

L'invention concerne l'utilisation d'une composition alimentaire comprenant des acides gras de type oméga-3 pour la prévention, la diminution ou la disparition des désordres liés à l'absorption d'alcool, notamment la gueule de bois.

Les oméga-3 sont des acides gras polyinsaturés dont les principaux représentants sont les suivants:
- l'acide α-linolénique ou ω₃α (18:3; ALA),
- l'acide éicosapentaénoïque (20:5; EPA) ou acide timnodonique,
- l'acide docosahexaénoïque (22:6; DHA) ou acide cervonique.

Les oméga-3 utilisés peuvent provenir de plusieurs sources alimentaires telles que :
- les algues, en particulier *Iridaea, Porphyra* et *Gymnogongrus* au Chili, *Hypnea musciformis* et *Meristotheca senegalensis, Arthrospira* ou *Schizochytrium,*
- les poissons gras tels que le saumon sauvage, le flétan, le hareng, le maquereau, les anchois et les sardines,
- l'huile de lin, l'huile de germe de blé, l'huile de soja, l'huile de chanvre, l'huile de cameline, l'huile de perilla, l'huile de colza, l'huile de noix, l'huile de krill,et
- certains légumes verts tels que la mâche, le chou, la laitue.....

Cependant la proportion des différents acides gras peut varier d'une source à l'autre.

Les désordres liés à l'absorption d'alcool sont divers et nombreux, cependant l'invention n'est pas un médicament et n'a pas pour objet le traitement de pathologies créées par l'alcool telles que l'hépatite ou la cirrhose du foie, le cancer des voies aérodigestives....

Elle a pour objet de traiter les désordres consécutifs à l'absorption d'alcool telle que la gueule de bois et notamment les symptômes associés à la gueule de bois tels que les maux de tête, les nausées, les vomissements, les vertiges, la dépression, l'irritabilité la baisse de concentration, la presse, la fatigue, la faiblesse, la souffrance, l'anorexie, la bouche sèche, les tremblements et les diarrhées.

Les dits symptômes liés à la gueule de bois sont nombreux et il est bien évident que la composition alimentaire va avoir une efficacité sur tout ou partie de ces symptômes, qu'il s'agisse de la prévention, c'est-à-dire l'empêchement de survenue de ces symptômes en cas de prise de la composition avant l'apparition des symptômes, ou de la diminution, c'est-à-dire une sensation plus faible d'un ou plusieurs symptômes, ou de la disparition d'un ou plusieurs symptômes, en fonction d'une part du moment de prise de la composition par rapport à la consommation d'alcool, mais également de la variation interindividuelle.

L'invention repose donc sur la constatation faite par les Inventeurs que l'administration d'oméga-3, utilisés habituellement pour la protection cardiovasculaire, l'arthrite et l'inflammation, ou la stabilisation de l'humeur, permettait également de lutter efficacement contre les symptômes de la gueule de bois.

Dans un mode de réalisation avantageux, l'invention concerne l'utilisation d'une composition alimentaire telle que définie ci-dessus, dans laquelle les oméga-3 proviennent d'huile de poisson.

Par huile de poisson, il faut notamment comprendre les huiles de poisson gras qui sont particulièrement riches en oméga-3.

Dans un mode de réalisation avantageux, l'invention concerne l'utilisation d'une composition alimentaire telle que définie ci-dessus, dans laquelle la concentration en oméga-3 est comprise de 0,1% (p/p) à 60% (p/p), préférentiellement de 0,1% (p/p) à 10% (p/p), plus préférentiellement de 1 % (p/p) à 5% (p/p), en particulier 2,2%, par rapport au poids total de la composition.

En deçà de 0,1% d'oméga-3, la proportion d'oméga-3 est trop faible pour observer une activité sur les symptômes de la gueule de bois.

Au delà de 60%, il n'y a plus de différence d'activité observée.

Dans un mode de réalisation avantageux, l'invention concerne l'utilisation d'une composition alimentaire telle que définie ci-dessus, dans laquelle les oméga-3 comprennent de l'acide eicosapentaenoïque (EPA) ou de l'acide docohexaenoïque (DHA).

Dans un mode de réalisation avantageux, l'invention concerne l'utilisation d'une composition alimentaire telle que définie ci-dessus, dans laquelle la proportion en EPA par rapport à la proportion totale en oméga-3 est comprise de 1% (p/p) à 99% (p/p), préférentiellement de 10% (p/p) à 90% (p/p), en particulier 51% (p/p) et la proportion en DHA par rapport à la proportion totale en oméga-3 est comprise de 99% (p/p) à 1% (p/p), préférentiellement de 90% (p/p) à 10% (p/p), en particulier 34% (p/p).

Dans un mode de réalisation avantageux, l'invention concerne l'utilisation d'une composition alimentaire telle que définie ci-dessus, dans laquelle la composition comprend de plus de l'acide gamma linolénique (GLA), provenant notamment d'huile de bourrache.

L'acide gamma linolénique est un acide gras essentiel qui fait partie des oméga-6.

L'acide gamma linolénique peut provenir de plusieurs sources alimentaires telles que l'huile de bourrache, l'huile de cassis, l'huile d'onagre, l'huile de tournesol, l'huile de carhame, l'huile de pépins de courge, ou la spiruline.

Avantageusement, le GLA provient de l'huile de bourrache, en particulier *Borago officinalis.*

Dans un mode de réalisation avantageux, l'invention concerne l'utilisation d'une composition alimentaire telle que définie ci-dessus, dans laquelle la concentration en huile de bourrache est comprise de 30% (p/p) à 99% (p/p), notamment de 40% (p/p) à 90% (p/p), en particulier 74% (p/p), par rapport au poids total de la composition.

En deçà de 30%, la proportion d'huile de bourrache est trop faible pour que la composition conserve son activité.

Dans un mode de réalisation avantageux, l'invention concerne l'utilisation d'une composition alimentaire telle que définie ci-dessus, dans laquelle la proportion en GLA par rapport à la proportion en huile de bourrache est comprise de 5% (p/p) à 60% (p/p), préférentiellement de 10% (p/p) à 50% (p/p), plus préférentiellement de 10% (p/p) à 30% (p/p), en particulier 22%.

Dans un mode de réalisation avantageux, l'invention concerne l'utilisation d'une composition alimentaire telle que définie ci-dessus, dans laquelle la composition comprend de plus:
de la vitamine B6 en proportion de 0,01% (p/p) à 0,5% (p/p), préférentiellement de 0,05%
(p/p) à 0,3% (p/p), en particulier 0,1% (p/p), par rapport au poids total de la composition, et constitue ainsi un complexe régulateur.

L'alcool en provoquant une inhibition de la Δ6-désaturase perturbe le métabolisme de GLA, EPA et DHA.

La vitamine B6 participe également au métabolisme des lipides.

Un autre avantage de l'invention est donc de fournir un complexe régulateur comprenant ou constitué de GLA, EPA, DHA et vitamines B6 permettant de réguler la teneur en GLA, EPA et DHA et par conséquent de lutter contre la gueule de bois.

Le complexe régulateur correspond donc à un complexe primaire permettant de lutter contre la gueule de bois.

Dans un mode de réalisation avantageux, l'invention concerne l'utilisation d'une composition alimentaire constituant un complexe régulateur, telle que définie ci-dessus, dans laquelle la composition comprend de plus un complexe anti-oxydant comprenant :
- de la vitamine C en proportion de 1% (p/p) à 20% (p/p), préférentiellement de 1% (p/p) à 10% (p/p), en particulier 6% (p/p), par rapport au poids total de la composition,
- de l'extrait de chardon marie, en particulier de l'extrait sec de chardon marie, en proportion de 1% (p/p) à 10% (p/p), préférentiellement de 2% (p/p) à 6% (p/p), en particulier 4% (p/p), par rapport au poids total de la composition,
- de l'extrait de figue de barbarie, en particulier de l'extrait sec de figue de barbarie, en proportion de 0,1% (p/p) à 5% (p/p), préférentiellement de 1% (p/p) à 5% (p/p), en particulier 3% (p/p), par rapport au poids total de la composition.

L'alcool provoque également un stress oxydatif et la peroxydation des lipides conduisant aussi à un catabolisme de GLA, EPA et DHA. Par conséquent, un complexe anti-oxydant comprenant ou constitué :
de vitamine C telle que définie ci-dessus, qui exerce une action anti-oxydante, combinée a un extrait de chardon marie (*Sylibum marianum*) qui exerce une action sur le foie, et combiné à un extrait de figue de barbarie (*Opuntia ficus indica*), aqueux ou huileux, qui possède également des propriétés anti-oxydantes,
permet de réduire le stress oxydatif et la peroxydation lipidique et restaurer ainsi la teneur en GLA, EPA et DHA.

Un autre avantage de l'invention est donc d'associer le complexe régulateur ci-dessus défini avec le complexe anti-oxydant et d'obtenir ainsi un effet synergique sur la restauration d'EPA, GLA et DHA et par conséquent sur le traitement de la gueule de bois.

Dans un mode de réalisation avantageux, l'invention concerne l'utilisation d'une composition alimentaire constituant un complexe régulateur, telle que définie ci-dessus, dans laquelle la composition comprend de plus un complexe correcteur comprenant
- du magnésium, en proportion de 1% (p/p) à 10% (p/p), en particulier 2,8% (p/p), par rapport au poids total de la composition,
- de la vitamine B1 en proportion de 0,01% (p/p) à 1% (p/p), préférentiellement de 0,1% (p/p) à 0,5% (p/p), en particulier 0,21 % (p/p), par rapport au poids total de la composition.

L'alcool provoque également une déficience en magnésium et en vitamine B1 qui vont également contribuer à l'apparition des symptômes de la gueule de bois.

Un complexe correcteur comprenant du magnésium, notamment sous forme d'oxyde de magnésium et de la vitamine B1 permet d'apporter les nutriments qui peuvent manquer lors d'une absorption excessive d'alcool et lutter ainsi contre les symptômes de la gueule de bois.

Le magnésium peut provenir d'autres sources, comme le chlorure de magnésium, le sulfate de magnésium... bien connues de l'homme du métier.

Un autre avantage de l'invention est donc d'associer le complexe régulateur ci-dessus défini avec le complexe correcteur et d'obtenir ainsi un effet synergique sur le traitement de la gueule de bois.

Dans un mode de réalisation avantageux, l'invention concerne l'utilisation d'une composition alimentaire constituant un complexe régulateur, telle que définie ci-dessus, dans laquelle la composition comprend de plus un complexe anti-oxydant tel que défini ci-dessus et un complexe correcteur tel que défini ci-dessus.

Encore un autre avantage de l'invention est d'associer le complexe régulateur ci-dessus défini avec le complexe anti-oxydant ci-dessus défini et le complexe correcteur ci-dessus défini et obtenir ainsi un effet synergique des trois complexes agissant chacun sur l'un des processus physiologiques perturbés et responsables des symptômes de la gueule de bois permettant ainsi la prévention, la diminution et/ou la disparition desdits symptômes.

En fonction des personnes consommant de l'alcool et de la quantité d'alcool absorbé, ainsi que de l'intensité des symptômes
il peut donc être administré une composition alimentaire comprenant ou constituée de:
soit le complexe régulateur seul,
soit une association complexe régulateur-complexe anti-oxydant,
soit une association complexe régulateur-complexe correcteur,
soit une association complexe régulateur- complexe anti-oxydant- complexe correcteur.

L'association des différents complexes et en particulier des trois complexes (complexe régulateur- complexe anti-oxydant- complexe correcteur) permet une potentialisation des effets de chaque complexe pris séparément.

La composition de l'invention peut être sous toute forme adaptée à une administration orale, en particulier sous forme de gélules, de comprimés, ou de capsules molles.

La composition comprenant les trois complexes est détaillée dans l'exemple 1.

Dans le cadre d'une consommation d'alcool, il est recommandé de prendre de 8,82 mg à 44,1 mg d'omega-3, entre 2h avant l'absorption d'alcool, et tant que l'alcoolémie est positive, soit de 1 à 5 capsules de la composition de l'exemple 1.

Selon un autre aspect, la présente invention concerne une composition telle que définie ci-dessus, comprenant ou étant constituée:
- d'oméga-3, lesdits oméga-3 étant en proportion comprise entre 0,1% (p/p) à 60% (p/p), préférentiellement de 0,1% (p/p) à 10% (p/p), plus préférentiellement de 1% (p/p) à 5% (p/p), en particulier 2,2%, par rapport au poids total de la composition.
- de GLA provenant notamment d'huile de bourrache, l'huile de bourrache étant en proportion de 30% (p/p) à 99% (p/p), notamment de 40% (p/p) à 90% (p/p), en particulier 74% (p/p), par rapport au poids total de la composition.

Dans un mode de réalisation avantageux, la présente invention concerne une composition telle que définie ci-dessus, dans laquelle lesdits oméga-3 comprennent de l'acide eicosapentaenoïque (EPA) en proportion par rapport à la proportion totale en oméga-3 est comprise de 1% (p/p) à 99% (p/p), préférentiellement de 10% (p/p) à 90% (p/p), en particulier 51% (p/p) et de l'acide docohexaenoïque (DHA) en proportion par rapport à la proportion totale en oméga-3 est comprise de 99% (p/p) à 1% (p/p), préférentiellement de 90% (p/p) à 10% (p/p), en particulier 34% (p/p).

Dans un mode de réalisation avantageux, la présente invention concerne une composition telle que définie ci-dessus, dans laquelle l'huile de bourrache comprend de l'acide gamma linolénique (GLA), la proportion en GLA par rapport à la proportion en huile de bourrache étant comprise de 5% (p/p) à 50% (p/p), préférentiellement de 10% (p/p) à 60% (p/p), plus préférentiellement de 10% (p/p) à 30% (p/p), en particulier 22%.

Dans un mode de réalisation avantageux, la présente invention concerne une composition telle que définie ci-dessus, comprenant de plus la vitamine B6 en proportion de 0,01% (p/p) à 0,5% (p/p), préférentiellement de 0,05% (p/p) à 0,3% (p/p), en particulier 0,1% (p/p), par rapport au poids total de la composition
constituant un complexe régulateur tel que défini précédemment..

Dans un mode de réalisation avantageux, la présente invention concerne une composition constituant un complexe régulateur, telle que définie ci-dessus, comprenant de plus un complexe anti-oxydant comprenant :
- de la vitamine C en proportion de 1% (p/p) à 20% (p/p), préférentiellement de 1% (p/p) à 10% (p/p), en particulier 6% (p/p), par rapport au poids total de la composition,,
- de l'extrait de chardon marie, en particulier de l'extrait sec de chardon marie, en proportion de 1% (p/p) à 10% (p/p), préférentiellement de 2% (p/p) à 6% (p/p), en particulier 4% (p/p), par rapport au poids total de la composition,
- de l'extrait de figue de barbarie, en particulier de l'extrait sec de figue de barbarie, en proportion de 0,1% (p/p) à 5% (p/p), préférentiellement de 1% (p/p) à 5% (p/p), en particulier 3% (p/p), par rapport au poids total de la composition

Dans un mode de réalisation avantageux, la présente invention concerne une composition constituant un complexe régulateur, telle que définie ci-dessus, comprenant de plus un complexe correcteur comprenant :
- du magnésium en proportion de 1% (p/p) à 10% (p/p), en particulier 2,8% (p/p), par rapport au poids total de la composition,
- de la vitamine B1 en proportion de 0,01% (p/p) à 1% (p/p), préférentiellement de 0,1 % (p/p) à 0,5% (p/p), en particulier 0,2 1 % (p/p), par rapport au poids total de la composition.

Dans un mode de réalisation avantageux, la présente invention concerne une composition constituant un complexe régulateur, telle que définie ci-dessus, comprenant de plus un complexe anti-oxydant tel que défini ci-dessus, et un complexe correcteur tel que défini ci-dessus.

### EXEMPLES:

### EXEMPLE 1 : Composition comprenant le complexe régulateur, le complexe anti-oxydant et le complexe correcteur

| **Complexe** | **Liste des ingrédients** | **Poids en mg par capsule** | **Teneur en actif pour 5 capsules** |
|---|---|---|---|
| **REGULATEUR** | Huile de graine de bourrache (*Borago officinalis*) 22% G.L.A. | 300,00 | 1500 mg d'huile de bourrache Dont 330 mg de G.L.A. |
| | Huile de poissons gras concentrée en acides gras (80%) titrés à 35% en Oméga 3 Dont 18% EPA Dont 12% DHA | 31,50 | 157,5 mg d'huile de poissons Dont 44,1 mg d'oméga3 Dont 22,7 mg d'E.P.A. Dont 15, mg de D.H.A. |
| | Vitamine B6 (Pyridoxine HCl) | 0,51* | 2 mg de vitamine B6 (Vit B6 sous forme de base) |
| **ANTI-OXYDANT** | Vitamine C (100% acide ascorbique) | 25,20* | 120 mg de vitamine C |
| | Extrait sec de fruit de chardon-marie (*Sylibum marianum*) 2% de sylimarine Solvant : acétone Support : maltodextrine | 16,00 | 80 mg d'extrait Dont 1,6 mg de sylimarine |
| | Extrait sec de figue de barbarie CactiNea© (*Opuntia ficus indica*) Solvant : eau | 12,00 | 60 mg d'extrait Dont 55 mg de polyphénols |
| **CORRECTEUR** | Vitamine B1 (Thiamine chlorhydrate) | 1,12* | 4,2 mg de vitamine B1 (Vit B1 sous forme de base) |
| | Oxyde de magnésium 60% Mg | 18,75 | 56,25 mg de magnésium |

| | | | |
|---|---|---|---|
| *surdosage de 5% pour les vitamines. Les vitamines sont surdosées pour compenser les pertes éventuelles en vitamines par dégradation de celles-ci. Les pourcentages indiqués dans la description ne tiennent pas compte du surdosage des vitamines. | | | |

La composition peut être, par exemple, sous forme de capsules molles de 702 mg ±7,5%. Dans ce cas, la capsule comprend la composition de l'exemple 1 (405mg) ainsi que la tunique et les adjuvants suivants :
Gélatine d'origine marine
Glycérine, Eau, Colorants
Cire blanche d'abeille
Lécithine de soja,
ladite tunique et lesdits adjuvants étant en quantité suffisante pour obtenir une capsule de 702mg ±7,5%.

### EXEMPLE 2 : Efficacité de la composition comprenant le complexe régulateur, le complexe anti-oxydant et le complexe correcteur sur les symptômes de la gueule de bois.

L'administration de la composition alimentaire de l'exemple 1 sur un panel de personnes, avant et après consommation d'alcool a permis de montrer, en auto-évaluation, une efficacité de la composition 1 sur les désordres liés à l'absorption d'alcool, notamment la gueule de bois. Des tests ont été effectués par un laboratoire indépendant pour estimer l'efficacité de la composition alimentaire de l'exemple 1, selon les modalités suivantes.

### Mise en oeuvre des tests :

Un étui de 15 capsules a été distribué à chaque panéliste. Les consignes d'utilisation du produit ont été fournies avec le questionnaire. Ce dernier a été envoyé directement sur la boite de messagerie électronique des consommateurs pour collecter leur avis.
- Température d'usage : Ambiante
- Modalités d'usage : 3 capsules avant la soirée et 2 au coucher

### Dispositions générales à l'étude :

Les tests ont été menés chez le consommateur. Le produit a été envoyé à son domicile par voie postale. Les résultats de 103 panélistes ont été exploités.
Chaque panéliste a été recruté par voie téléphonique à partir du panel base du laboratoire. Les fréquences de participation aux tests de chaque consommateur respecte la norme V09-500.

Age : 50% de 25 à 30 ans, 25% de 31 à 35 ans et 25 % de 36 à 40 ans.
Sexe : 21% d'hommes, 79% de femmes.

### Méthodologie :

Chaque panéliste a reçu sur sa boite e-mail un questionnaire individuel auto administré.
Au lendemain de la soirée, lorsque l'alcoolémie a été considérée nulle, les consommateurs ont renseigné leur satisfaction du produit grâce à un questionnaire.
La conduite de l'épreuve a suivi la norme NF-V09-500.

### Résultats après utilisation :

Les consommateurs devaient décrire comment ils se sentaient au lendemain de la prise du produit et comment ils se seraient senti s'ils n'avaient pas pris le produit en notant les sensations suivantes (0 correspond à une sensation pas du tout ressentie et 10 énormément ressentie).
La comparaison statistique des résultats a été obtenue par un test de Student. Avec le produit, toutes les sensations ont été perçues de façon significativement plus faible que sans la prise de produit.

Résultats de la question 'Diriez-vous que le produit réduit la sensation d'inconfort au lendemain d'une consommation d'alcool : 32% de tout à fait d'accord, 56% d'accord, 9% pas d'accord et 3% pas du tout d'accord.
88% des consommateurs ont considéré que le produit réduit la sensation d'inconfort au lendemain d'une consommation d'alcool.

Résultats de la question « Diriez-vous que le produit est efficace contre : »
Les consommateurs devaient répondre à différentes questions avec comme choix de réponse « je ne suis pas du tout d'accord » à « je suis tout à fait d'accord » sur une échelle de catégories à 4 points.
Le tableau présente les pourcentages par catégorie ainsi que l'éventuelle significativité des réponses favorables par rapport aux appréciations défavorables (lettres : S- significativement négatif, NS non significatif ou S+ significativement positif). Le test statistique qui a été utilisé suit une loi binomiale de paramètre p=1/2.
Globalement, les appréciations sur l'efficacité perçue du produit ont été assez ou très favorables. Seule l'efficacité contre le rythme cardiaque élevé a recueilli des appréciations favorables cumulées qui sont statistiquement non significatives.

Le tableau suivant liste différents critères ressentis :

| **Affirmations** | **pas du tout d'accord %** | **pas d'accord %** | **d'accord %** | **tout à fait d'accord %** | % **d'intentions favorables cumulées** |
|---|---|---|---|---|---|
| **Sensation de soif** | 6% | 21% | 53% | 20% | 73% S+ |
| **Fatigue** | 9% | 19% | 55% | 17% | 72% S+ |
| **Maux de tête** | 6% | 13% | 54% | 27% | 82% S+ |
| **Vertiges** | 10% | 19% | 60% | 11% | 71% S+ |
| **Manque d'appétit** | 10% | 29% | 52% | 9% | 61% S+ |
| **Maux d'estomac** | 8% | 23% | 52% | 17% | 69% S+ |
| **Nausées** | 9% | 15% | 59% | 17% | 77% S+ |
| **Rythme cardiaque élevé** | 17% | 25% | 46% | 12% | 57% NS |
| **Difficulté de concentration** | 13% | 21% | 54% | 12% | 66% S+ |
| **Sensation globale de gueule de bois** | 5% | 9% | 59% | 27% | 86% S+ |

## Revendications

1. Utilisation d'une composition alimentaire comprenant :
- des acides gras de type oméga-3,
- de l'acide gamma linolénique (GLA), provenant notamment d'huile de bourrache,
- de la vitamine B6 en proportion de 0,01% (p/p) à 0,5% (p/p), préférentiellement de 0,05% (p/p) à 0,3% (p/p), en particulier 0,1% (p/p), par rapport au poids total de la composition,
constituant ainsi un complexe régulateur,
pour la prévention, la diminution ou la disparition des désordres liés à l'absorption d'alcool, notamment la gueule de bois.

2. Utilisation selon la revendication 1, dans laquelle les oméga-3 proviennent d'huile de poisson.

3. Utilisation selon la revendication 2, dans laquelle la concentration en oméga-3 est comprise de 0,1% (p/p) à 60% (p/p), préférentiellement de 0,1 % (p/p) à 10% (p/p), plus préférentiellement de 1% (p/p) à 5% (p/p), en particulier 2,2%, par rapport au poids total de la composition.

4. Utilisation selon la revendication 2 ou 3, dans laquelle les oméga-3 comprennent de l'acide eicosapentaenoïque (EPA) ou de l'acide docohexaenoïque (DHA).

5. Utilisation selon la revendication 4, dans laquelle la proportion en EPA par rapport à la proportion totale en oméga-3 est comprise de 1% (p/p) à 99% (p/p), préférentiellement de 10% (p/p) à 90% (p/p), en particulier 51% (p/p) et la proportion en DHA par rapport à la proportion totale en oméga-3 est comprise de 99% (p/p) à 1% (p/p), préférentiellement de 90% (p/p) à 10% (p/p), en particulier 34% (p/p).

6. Utilisation selon l'une des revendications 1 à 5, dans laquelle la concentration en huile de bourrache est comprise de 30% (p/p) à 99% (p/p), notamment de 40% (p/p) à 90% (p/p), en particulier 74% (p/p, par rapport au poids total de la composition.

7. Utilisation selon la revendication 6, dans laquelle la proportion en GLA par rapport à la proportion en huile de bourrache est comprise de 5% (p/p) à 60% (p/p), préférentiellement de 10% (p/p) à 50% (p/p), plus préférentiellement de 10% (p/p) à 30% (p/p), en particulier 22%.

8. Utilisation selon l'une des revendications 1 à 7, dans laquelle la composition comprend de plus un complexe anti-oxydant comprenant :
- de la vitamine C en proportion de 1 % (p/p) à 20% (p/p), préférentiellement de 1 % (p/p) à 10% (p/p), en particulier 6% (p/p), par rapport au poids total de la composition,
- de l'extrait de chardon marie, en particulier de l'extrait sec de chardon marie, en proportion de 1 % (p/p) à 10% (p/p), préférentiellement de 2% (p/p) à 6% (p/p), en particulier 4% (p/p), par rapport au poids total de la composition,
- de l'extrait de figue de barbarie, en particulier de l'extrait sec de figue de barbarie, en proportion de 0,1% (p/p) à 5% (p/p), préférentiellement de 1% (p/p) à 5% (p/p), en particulier 3% (p/p), par rapport au poids total de la composition.

9. Utilisation selon l'une des revendications 1 à 7, dans laquelle la composition comprend de plus un complexe correcteur comprenant :
du magnésium, en proportion de 1% (p/p) à 10% (p/p), en particulier 2,8% (p/p), par rapport au poids total de la composition,
de la vitamine B1 en proportion de 0,01% (p/p) à 1% (p/p),
préférentiellement de 0,1% (p/p) à 0,5% (p/p), en particulier 0,21% (p/p) par rapport au poids total de la composition.

10. Utilisation selon l'une des revendications 1 à 7, dans laquelle la composition comprend de plus un complexe anti-oxydant tel que défini dans la revendication 8 et un complexe correcteur tel que défini dans la revendication 9.

11. Composition telle que définie dans la revendication 1, comprenant ou étant constituée :
- d'oméga-3, lesdits oméga-3 étant en proportion comprise entre 0,1% (p/p) à 60% (p/p), préférentiellement de 0,1% (p/p) à 10% (p/p), plus préférentiellement de 1 % (p/p) à 5% (p/p), en particulier 2,2%, par rapport au poids total de la composition.
- de GLA provenant notamment d'huile de bourrache, l'huile de bourrache étant en proportion de 20% (p/p) à 99% (p/p), notamment de 40% (p/p) à 90% (p/p), en particulier 74% (p/p, par rapport au poids total de la composition.
- de la vitamine B6 en proportion de 0,01% (p/p) à 0,5% (p/p), préférentiellement de 0,05% (p/p) à 0,3% (p/p), en particulier 0,1% (p/p), par rapport au poids total de la composition,
constituant un complexe régulateur.

12. Composition selon la revendication 11, dans laquelle lesdits oméga-3 comprennent de l'acide eicosapentaenoïque (EPA) en proportion par rapport à la proportion totale en oméga-3 est comprise de 1 % (p/p) à 99% (p/p), préférentiellement de 10% (p/p) à 90% (p/p), en particulier 51% (p/p) et de l'acide docohexaenoïque (DHA) en proportion par rapport à la proportion totale en oméga-3 est comprise de 99% (p/p) à 1 % (p/p), préférentiellement de 90% (p/p) à 10% (p/p), en particulier 34% (p/p).

13. Composition selon la revendication 11 ou 12, dans laquelle l'huile de bourrache comprend de l'acide gamma linolénique (GLA), la proportion en GLA par rapport à la proportion en huile de bourrache étant comprise de 5% (p/p) à 50% (p/p), préférentiellement de 10% (p/p) à 60% (p/p), plus préférentiellement de 10% (p/p) à 30% (p/p), en particulier 22%.

14. Composition selon l'une des revendications 11 à 13, comprenant de plus un complexe anti-oxydant comprenant :
- de la vitamine C en proportion de 1% (p/p) à 20% (p/p), préférentiellement de 1% (p/p) à 10% (p/p), en particulier 6% (p/p), par rapport au poids total de la composition,
- de l'extrait de chardon marie, en particulier de l'extrait sec de chardon marie, en proportion de 1% (p/p) à 10% (p/p), préférentiellement de 2% (p/p) à 6% (p/p), en particulier 4% (p/p), par rapport au poids total de la composition,
- de l'extrait de figue de barbarie, en particulier de l'extrait sec de figue de barbarie, en proportion de 0,1% (p/p) à 5% (p/p), préférentiellement de 1% (p/p) à 5% (p/p), en particulier 3% (p/p), par rapport au poids total de la composition.

15. Composition selon l'une des revendications 11 à 13, comprenant de plus un complexe correcteur comprenant :
- du magnésium, en proportion de 1% (p/p) à 10% (p/p), en particulier 2,8% (p/p), par rapport au poids total de la composition,
- de la vitamine B1 en proportion de 0,01% (p/p) à 1% (p/p), préférentiellement de 0,1% (p/p) à 0,5% (p/p), en particulier 0,21% (p/p) par rapport au poids total de la composition.

16. Composition selon l'une des revendications 11 à 13, comprenant de plus un complexe anti-oxydant tel que défini dans la revendication 14 et un complexe correcteur tel que défini dans la revendication 15.
